# EUROPÄISCHE PATENTSCHRIFT

(11) **EP 2 711 080 B1**
(45) Veröffentlichungstag und Bekanntmachung des Hinweises auf die Patenterteilung: **10.04.2019**
(21) Anmeldenummer: 12185613.2
(22) Anmeldetag: 24.09.2012
(51) Int. Cl.: B01L 3/02, A61M 5/32, G01N 35/10

(54) **HOHLNADEL FÜR EINEN PROBENPIPETTOR**
HOLLOW NEEDLE FOR A SAMPLE PIPETTOR
AIGUILLE CREUSE POUR PIPETTE DE PRÉLÈVEMENT

(43) Veröffentlichungstag der Anmeldung: 26.03.2014
(73) Patentinhaber: Siemens Healthcare Diagnostics Products GmbH, 35041 Marburg (DE)
(72) Erfinder: Kressirer, Rudolf, 65779 Kelkheim (DE); Ziechner, Eike, 61273 Wehrheim (DE)

(56) Entgegenhaltungen:
- WO-A1-2004/064903
- WO-A1-2009/024522
- WO-A1-2009/060047
- US-A- 2 717 599
- US-A- 6 135 172

## Beschreibung

Die Erfindung betrifft eine Hohlnadel für einen Probenpipettor in einem automatischen Analysegerät, der geeignet ist, Probenflüssigkeit aus einem verschlossenen Probengefäß zu entnehmen, indem der Deckel oder die Kappe des Probengefäßes mit der Hohlnadel durchstoßen wird.

Zahlreiche Nachweis- und Analyseverfahren zur Bestimmung physiologischer Parameter in Körperflüssigkeitsproben oder anderer Parameter in anderen biologischen Proben werden heute automatisiert in großer Anzahl in entsprechenden automatischen Analysegeräten durchgeführt. Im Bereich der Labordiagnostik werden die zu analysierenden Körperflüssigkeitsproben, wie Blut, Plasma, Serum oder Urin, in verschlossenen Probengefäßen zur Verfügung gestellt. Die Probengefäße werden dem Analysegerät einzeln oder gruppenweise in geeigneten Gestellen angeordnet zugeführt. Üblicherweise werden die Probengefäße mittels eines Transportsystems zunächst an einer Identifikationseinrichtung vorbeigeführt, die Informationen zur Identität der Probe, die auf dem Probengefäß angebracht sind, z.B. in Form eines Barcodes, liest und an eine Speichereinheit weiterleitet. Sodann wird jedem Probengefäß ein Aliquot der Probenflüssigkeit entnommen und in ein Reaktionsgefäß transferiert, in welchem dann das analytische Testverfahren durchgeführt wird.

Körperflüssigkeitsproben befinden sich üblicherweise in Probengefäßen aus Kunststoff, seltener aus Glas, die mit einem Deckel oder einer Kappe aus Kunststoff oder mit bis zu 1 cm dicken Gummistopfen verschlossenen sind. Blut-, Plasma- und Serumproben werden dem Analysegerät bevorzugterweise in den Blutentnahmeröhrchen zugeführt. Blutentnahmeröhrchen bestehen üblicherweise aus transparentem Kunststoff, und die Verschlussvorrichtung ist mit einem speziellen Anschluss für Kanülen ausgestattet. Blutentnahmeröhrchen sind dabei (außer beim so genannten Sarstedtprinzip) häufig als Unterdrucksysteme ausgestaltet: Innerhalb des Probengefäßes dieses Typs herrscht von vornherein ein Unterdruck. Wird es auf den mit der Punktionskanüle verbundenen Adapter gesteckt, wird durch diesen Unterdruck das Blut angesaugt. Ein Vorteil dieses Systems ist, dass die angesaugte Blutmenge vergleichsweise konstant ist und somit auch die Menge eines vorab in das Blutentnahmeröhrchen eingebrachten Gerinnungshemmers (z.B. Citrat, EDTA, Heparin) genau bemessen werden kann. Die Blutentnahmeröhrchen sind üblicherweise zur Erhaltung des Drucks mit einem elastischen Verschluss abgedichtet.

Zur Entnahme von Probenflüssigkeit aus den Probengefäßen und zum Transfer von Probenflüssigkeit in ein Reaktionsgefäß umfasst ein automatisches Analysegerät einen Probenpipettor mit einer Hohlnadel. Die Hohlnadel ist an einem Transportarm befestigt und kann so zwischen mindestens einer Probenentnahmeposition und mindestens einer Probenabgabeposition bewegt werden. An der Probenentnahmeposition wird die Hohlnadel möglichst entlang der Mittelachse des Probengefäßes vertikal nach unten bewegt bis die Nadelspitze in die Probenflüssigkeit eintaucht. Das Eintauchen wird mit Hilfe eines entsprechenden Sensors registriert. Durch Erzeugen eines Unterdrucks in der Hohlnadel wird Probenflüssigkeit angesaugt, die Hohlnadel wird vertikal nach oben und anschließend horizontal zur Probenabgabeposition bewegt. An der Probenabgabeposition wird dann eine definierte Probenmenge in ein Reaktionsgefäß gegeben. Bekannte Hohlnadeln für derartige Probenpipettoren bestehen häufig aus Edelstahl und haben eine im Wesentlichen zylinderförmige Grundform mit einem zentralen Hohlkanal, wobei die Hohlnadel axiale Abschnitte mit variierenden Innen- und Außenradien aufweisen kann.

Soll die Probenentnahme aus verschlossenen Probengefäßen erfolgen, muss der Probenpipettor samt Hohlnadel so ausgelegt sein, dass die vertikale Abwärtsbewegung der Hohlnadel mit einer solchen Kraft durchgeführt wird, dass die Verschlussvorrichtung des Probengefäßes durchstoßen werden kann. Gleichzeitig muss jedoch sicher gestellt sein, dass die Hohlnadel nicht beschädigt wird, da es sonst zu Fehlern bei der Probenaufnahme oder -abgabe kommen könnte.

Um den Kraftaufwand zum Durchstoßen einer Verschlussvorrichtung eines Probengefäßes möglichst gering zu halten, sind dafür vorgesehene Hohlnadeln vergleichsweise massiv ausgeführt und üblicherweise angespitzt. EP-B1-1420255 (Figuren 34-37, Absätze 0111-0118) beschreibt beispielsweise eine Hohlnadel, die an der Spitze pyramidenförmig oder konisch ausgeformt ist, so dass ein Scheitelpunkt entsteht, an dem die Kraft bei der Abwärtsbewegung der Hohlnadel gebündelt wird und eine Verschlussvorrichtung eines Probengefäßes, z.B. eine Gummikappe, mit vergleichsweise geringem Kraftaufwand durchstoßen werden kann.

Ein weiteres Problem beim Durchstechen von Verschlussvorrichtungen mit der Hohlnadel eines Probenpipettors besteht in der Stanzmesserwirkung der Nadel, die dazu führt, dass Teile der perforierten Verschlussvorrichtung, z.B. Gummikrümel, das Pipettierloch der Nadel verstopfen können. Dieses Problem wird unter anderem in DE-T2-69827465 (US 6,135,172) dadurch gelöst, dass der Hohlkanal nicht an der Spitze selbst, sondern seitlich am Nadelkörper nach außen mündet.

Weitere bekannte Hohlnadeln sind in WO2004/064903 und WO2009/024522 offenbart.

Noch ein weiteres Problem besteht darin, dass Fehler bei der Probenaufnahme insbesondere dadurch entstehen, dass die Nadel beim Kontakt mit dem Verschlusselement eines Probengefäßes trotz sorgfältiger Justierung seitlich ausgelenkt wird und nicht wie gewünscht entlang oder zumindest parallel zur Mittelachse des Probengefäßes, sondern schräg durch das Verschlusselement stößt. Im schlimmsten Fall kann dies dazu führen, dass die Nadel die Innenwand des Probenröhrchens berührt und eventuell sogar zerstört, wodurch die Probe und/oder die Nadel unbrauchbar und das Gerät eventuell kontaminiert werden können. Weiterhin wurde beobachtet, dass das Sensorsignal für das Eintauchen in die Probe ausgelöst werden kann, wenn die Nadel die Innenwand des Probengefäßes berührt, obwohl ein tatsächliches Eintauchen noch nicht stattgefunden hat. Dies erhöht die Gefahr, dass Luft anstelle von Probenflüssigkeit pipettiert wird.

Der vorliegenden Erfindung lag also die Aufgabe zugrunde, einen Probenpipettor bereit zu stellen, der eine vergleichsweise wenig fehleranfällige Entnahme von Probenflüssigkeit aus einem verschlossenen Probengefäß ermöglicht.

Diese Aufgabe wird durch eine Hohlnadel, welche gemäß Anspruch 1 ausgebildet ist, gelöst.

Es wurde gefunden, dass die unerwünschte seitliche Auslenkung der Hohlnadel eines Probenpipettors dadurch entsteht, dass beim Durchstoßen eines Verschlussstopfens Querkräfte in radialer Richtung auf die Hohlnadel wirken. Dadurch wird die Hohlnadel beim weiteren Eintauchen in das Probenröhrchen aus der Vertikalen ausgelenkt, so dass die Innenwand berührt wird und die Gefahr einer Luftpipettierung besteht. Die Querkräfte entstehen insbesondere, wenn die Nadelspitze asymmetrisch ausgebildet ist.

Gemäß der vorliegenden Erfindung wird der Entstehung von Querkräften durch entsprechenden Anschliff der Nadelspitze entgegengewirkt. Durch Anschliffe der Nadelspitze entstehen hier ebene Flächen, wobei die jeweiligen Flächen hinsichtlich ihres Winkels zur Achse der Hohlnadel und der Größe ihrer Oberfläche so eingebracht werden, dass sich die bei einer axialen Bewegung in ein elastisches Material resultierenden radialen Kraftkomponenten gegenseitig aufheben. Dadurch wird ein Auslenken der Hohlnadel beim Eintauchen in das Blutentnahmeröhrchen vermieden.

Gegenstand der vorliegenden Erfindung ist daher eine Hohlnadel für einen Probenpipettor, wobei die Hohlnadel eine im Wesentlichen gerade zylindrische Grundform mit einer Spitze zum Durchstoßen einer Verschlussvorrichtung aufweist, und die im Bereich der Spitze mindestens vier Flächen derart aufweist, dass sich die an den jeweiligen Flächen bei einer axialen Bewegung in ein elastisches Material resultierenden radialen Kraftkomponenten gegenseitig aufheben.

Erfindungsgemäß weist die Hohlnadel im Bereich der Spitze zwei Flächen auf, deren jeweilige Normale mit der Achse der Hohlnadel in einer Ebene liegen. Mit anderen Worten: Es wird zu einer bestehenden Fläche eine zweite Fläche geschaffen, deren Normale so ausgerichtet ist, dass sie die Querkräfte der bestehenden Fläche zumindest teilweise direkt auszugleichen vermag. Hierzu werden die Oberfläche und der Neigungswinkel der Fläche entsprechend gewählt. Hierdurch ist es möglich eine Ausgleichsfläche für einen schrägen Anschliff zu schaffen, die die radialen Kräfte der ersten Fläche des schrägen Anschliffs ausgleicht.

Vorteilhafterweise sind die Flächen derart ausgestaltet, dass eine vorgegebene Mindestwanddicke der Hohlnadel nicht unterschritten wird. Trotz der elastischen Eigenschaften der häufig verwendeten Gummiverschlüsse ist die Hohlnadel eines Probenpipettors aufgrund der hohen Pipettierfrequenz in modernen Analysegeräten einem erheblichen Verschleiß unterworfen, so dass die Hohlnadel als Verbrauchsmaterial anzusehen ist und regelmäßig gewechselt werden muss. Eine übermäßige Reduzierung der Wanddicke würde die Stabilität der Nadelspitze beeinträchtigen und die Lebensdauer reduzieren. Zusätzlich würden durch zu starke Reduzierung der Wandflächen Öffnungen an der Hohlnadel entstehen, an Stellen wo dies nicht gewünscht ist.

Erfindungsgemäß ist die Hohlnadel im Bereich der Spitze gebogen. Dadurch ergibt sich eine Biegung des Hohlkanals der Nadel, so dass der Austritt des Hohlkanals bei entsprechendem Anschnitt seitlich und nicht in axialer Richtung angeordnet ist. Eine der Flächen ist dann derart eingebracht, dass sie den Austritt des Hohlkanals der Hohlnadel umschließt.

Dadurch lässt sich die Spitze der Hohlnadel weiter in Richtung der Achse der Hohlnadel positionieren, so dass eine vergleichsweise symmetrischere Ausgestaltung und daher ein besserer Ausgleich der Querkräfte ermöglicht wird.

Die Hohlnadel ist zylindrisch ausgeformt, bevorzugterweise kreiszylindrisch, viereckig oder prismatisch. In einer besonders bevorzugten Ausführungsform ist der Querschnitt des Hohlnadelzylinders ein Viereck oder ein Rechteck mit konkav geschliffenen Seiten. Dies hat den Vorteil, dass die bei einer axialen Bewegung der Hohlnadel in ein elastisches Material entstehende Gleit- und Haftreibung vermindert ist, wodurch die Hohlnadel mit einem verhältnismäßig geringeren Kraftaufwand durch einen Gummistopfen hindurchgestoßen und wieder aus ihm herausgezogen werden kann.

Wie bereits beschrieben liegt die jeweilige Normale zweier der Flächen mit der Achse der Hohlnadel dabei in einer Ebene. Weiterhin ist die Hohlnadel im Bereich der Spitze gebogen und eine der Flächen umschließt den Austritt des Hohlkanals der Hohlnadel zumindest teilweise.

In zusätzlicher vorteilhafter Ausgestaltung umfasst die Hohlnadel einen sich verbreiternden axialen Abschnitt, der eine axiale Vertiefung aufweist. Da die Menge des entnommenen Aliquots aus dem Blutentnahmeröhrchen mittels Druck und/oder Unterdruck gesteuert wird, ist es zur Einhaltung der vorgegebenen Menge notwendig, einen Druckausgleich des Innenraums des Röhrchens mit der Umgebung herzustellen, da sonst durch einen entstehenden Unterdruck beim Pipettieren zu wenig Probenmenge entnommen wird. Durch einen sich verbreiternden Abschnitt wird die Perforationsöffnung beim Eintauchen der Hohlnadel geweitet. Die axiale Vertiefung ist dabei länger als die Dicke des Verschlusses, so dass sie vom Innenraum bis in den Außenraum reicht. Die Breite und Tiefe der Vertiefung sind so bemessen, dass der elastische Gummi nicht vollständig in die Vertiefung eindringt. So entsteht ein Luftkanal, der Innen- und Außenraum verbindet und einen Druckausgleich beim Eintauchen und während des Pipettierens erreicht.

Ein weiterer Gegenstand der vorliegenden Erfindung ist ein Probenpipettor, der eine erfindungsgemäße Hohlnadel umfasst. Ein erfindungsgemäßer Probenpipettor umfasst vorzugsweise ferner eine Halterung, mit der die Hohlnadel an einem zwischen mindestens einer Probenentnahmeposition und mindestens einer Probenabgabeposition beweglichen Transferarm angebracht ist. Ferner ist die Hohlnadel mit einem Antrieb verbunden, der die Hohlnadel vertikal bewegen kann. Bevorzugterweise weist der Probenpipettor einen Sensor zur Detektion des Füllstands der Probenflüssigkeit in dem Probengefäß auf.

Noch ein weiterer Gegenstand der vorliegenden Erfindung betrifft ein automatisches Analysegerät mit einem Probenpipettor, der eine erfindungsgemäße Hohlnadel umfasst. Erfindungsgemäße automatische Analysegeräte weisen mindestens ein Messsystem auf. Besonders verbreitet sind Messsysteme, die auf photometrischen (z.B. turbidimetrischen, nephelometrischen, fluorometrischen oder luminometrischen) oder radiometrischen Messprinzipien beruhen. Bevorzugterweise weist ein automatisches Analysegerät ferner eine Speichereinheit auf, die die Messergebnisse, die von dem Messsystem erfasst wurden, speichert. Das Analysegerät umfasst ferner ein Ausgabemedium, wie z.B. einen Monitor, einen Drucker oder eine Netzwerkverbindung, so dass die probenspezifischen Messwerte einem Benutzer zugänglich gemacht werden können.

Die Erfindung betrifft ferner ein Verfahren zum Herstellen einer Hohlnadel zum Durchstoßen einer Verschlussvorrichtung eines Probengefäßes gemäß Anspruch 6. Dazu werden im Bereich der Spitze einer Hohlnadel, die im Wesentlichen eine gerade zylindrische Grundform aufweist, mindestens vier Flächen hinsichtlich ihrer Winkel zur Achse der Hohlnadel und der Größe ihrer Oberflächen derart eingebracht, dass sich die an den jeweiligen Flächen bei einer axialen Bewegung in ein elastisches Material resultierenden radialen Kraftkomponenten gegenseitig aufheben.

Bevorzugterweise werden die mindestens vier Flächen durch Anschleifen der Oberfläche im Bereich der Spitze eingebracht.

Die Erfindung wird anhand einer Zeichnung näher erläutert. Darin zeigen:
- FIG 1: eine Aufsicht einer Hohlnadel aus Richtung des seitlichen Austritts des Hohlkanals,
- FIG 2: einen Schnitt durch die Hohlnadel,
- FIG 3: eine Aufsicht des Bereichs der Spitze der Hohlnadel in axialer Richtung,
- FIG 4: eine seitliche Aufsicht des Bereichs der Spitze der Hohlnadel,
- FIG 5: einen Schnitt durch den Bereich der Spitze der Hohlnadel in radialer Richtung,
- FIG 6: eine Aufsicht des Bereichs der Spitze der Hohlnadel aus entgegengesetzter Richtung gegenüber FIG 1, und
- FIG 7: Versuchsergebnisse zur Messung des Kräfteausgleichs.

Gleiche Teile sind in allen Figuren mit denselben Bezugszeichen versehen.

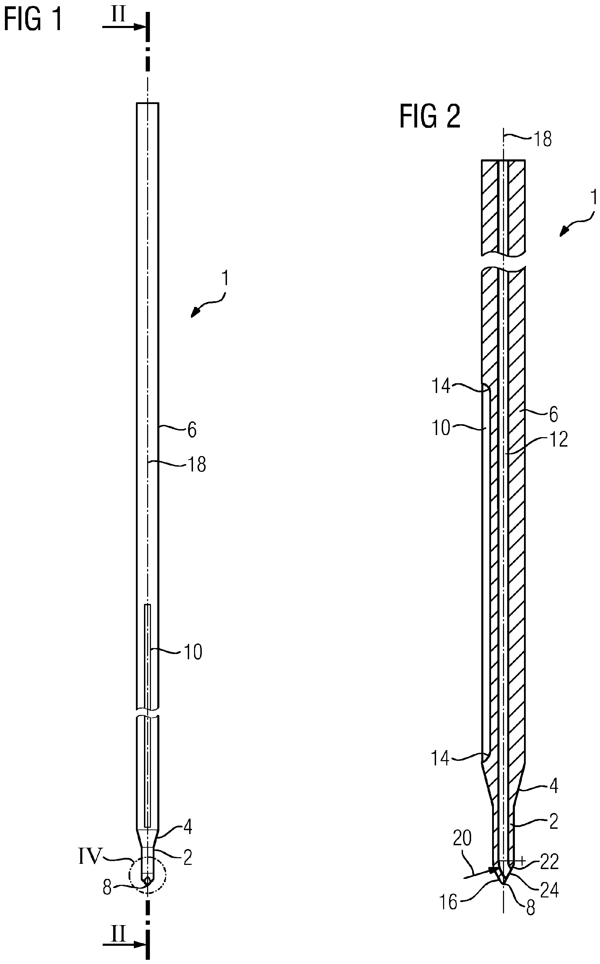

Die FIG 1 zeigt eine Hohlnadel 1 in Aufsicht. Die Hohlnadel 1 ist im Wesentlichen kreiszylinderförmig ausgestaltet und weist mehrere axiale Abschnitte 2, 4, 6 ausgehend von ihrer Spitze 8 auf. Andere Zylindergrundformen sind hier möglich, die Kreiszylinderform hat aber bestes Widerstandsmoment in alle Richtungen. An die Spitze 8 schließt sich ein erster axialer Abschnitt 2 an, der zur Bildung der Spitze 8 angeschliffen ist und im Folgenden noch weiter erläutert wird. Der erste axiale Abschnitt 2 weist einen Außendurchmesser von 1,2 mm auf.

An den Abschnitt 2 schließt sich ein kegelabschnittsförmiger zweiter axialer Abschnitt 4 an. Der Abschnitt 4 verbreitert den Außendurchmesser linear auf 2,1 mm. An den zweiten Abschnitt 4 schließt sich ein vergleichsweise langer Abschnitt 6 an, der einen konstanten Außendurchmesser von 2,1 mm aufweist. Im an den zweiten Abschnitt angrenzenden Bereich ist eine sich in axialer Richtung erstreckende schmale Vertiefung 10 eingebracht. Diese erstreckt sich über einen vergleichsweise weiten Teil des langen Abschnitts 6 und bildet beim Durchstoßen eines Verschlusses eines Blutentnahmeröhrchens einen Luftkanal, der Innen- und Außenraum des Röhrchens verbindet und einen Druckausgleich herbeiführt.

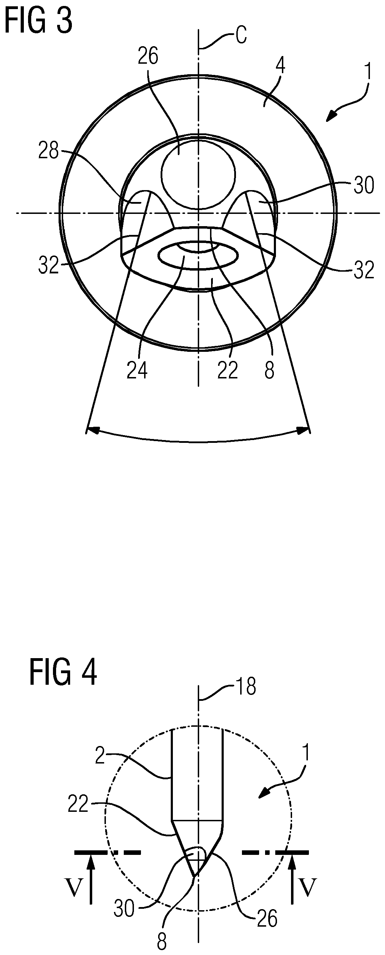

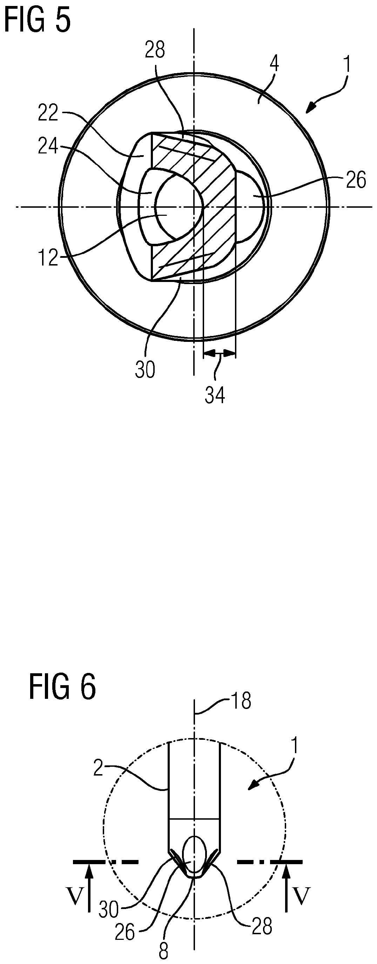

FIG 1 zeigt die Schnittebene II-II, die der Schnittzeichnung in FIG 2 entspricht. Der Bereich IV ist in den FIG 4 und FIG 6 vergrößert dargestellt.

FIG 2 zeigt neben den in FIG 1 gezeigten Merkmalen insbesondere das Profil der Vertiefung 10 und des Hohlkanals 12 in der Hohlnadel 1. Die Länge der Vertiefung 10 in axialer Richtung beträgt 134,07 mm, wobei die Vertiefung an ihren axialen Enden 14 ein viertelkreisförmiges Profil hat, das durch den Fertigungsprozess bedingt ist. Die Vertiefung 10 hat eine Tiefe in radialer Richtung von 0,5 mm.

Der Hohlkanal 12 hat über die gesamte Länge der Hohlnadel 1 einen konstanten Innendurchmesser von 0,6 mm. Im Bereich der Spitze 8 ist eine Biegung 16 des Hohlkanals 12 gezeigt, die bezogen auf die Achse 18 einen Biegeradius 20 von 1,15 mm aufweist. Im Fertigungsprozess wird die Hohlnadel 1 zunächst durch Ziehen in die im wesentlichen kreiszylindrische Form mit den zwei verschiedenen Durchmessern plus Innendurchmesser gebracht, anschließend in der Biegung 16 umgebogen und an der Fläche 22 abgeschliffen. Daraus resultiert eine gegen die Achse 18 derart geneigte Fläche 22, dass die Spitze 8 nahezu auf der Achse 18 liegt. Die Fläche 22 umschließt den Austritt 24 des Hohlkanals 12. Ein flacherer Anschnitt der Fläche 22 birgt die Gefahr des Abschneidens von Gummimaterial und Verstopfens des Hohlkanals 12 beim Durchstoßen des Verschlusses.

Im Bereich der Spitze 8 sind nun weitere Flächen eingebracht, die hinsichtlich Neigung und Oberfläche derart gewählt sind, dass sich die durch die jeweiligen Flächen entstehenden Kraftkomponenten in radialer Richtung gegenseitig ausgleichen. Die eingebrachten Flächen sind in den FIGs 3 bis 6 im Detail dargestellt. Hierbei ist darauf zu verweisen, dass die im Ausführungsbeispiel gezeigten Flächen lediglich eine bevorzugte Ausführungsform darstellen und hinsichtlich Anzahl, Neigung und Oberflächengröße variiert werden können, solange sie das Kriterium des Kraftausgleichs erfüllen.

FIG 3 zeigt eine axiale Aufsicht der Hohlnadel 1 mit der Spitze 8, der Fläche 22 und dem Austritt 24 des Hohlkanals 12 (siehe FIG 5). Die Anordnung der Flächen ist spiegelsymmetrisch bezüglich der Symmetrieebene C. Neben der Fläche 22 ist eine zweite Fläche 26 angeschliffen, die ebenfalls zur Achse 18 hin geneigt ist. Die im jeweiligen Mittelpunkt der Flächen 22, 26 gedachten Normalen der Flächen 22, 26 liegen mit der Achse 18 in einer Ebene. Die Fläche 26 selbst hat eine kleinere Oberfläche als die Fläche 22. Eine Vergrößerung der Fläche 26 durch tieferes Anschleifen würde die Wanddicke unterhalb der Fläche 26 übermäßig verringern, so dass ein stärkerer Ausgleich der Querkräfte hier nicht möglich ist.

Stattdessen sind zwei symmetrisch angeordnete, halbkreisförmige weitere Flächen 28, 30 seitlich angebracht. Neben der Neigung zur Achse 18 hin sind die Flächen 28, 30 auch derart geneigt, dass sich die Schnittlinien 32 mit der radial-azimutalen Ebene auf der vom Austritt 24 abgewandten Seite der Achse 18 schneiden und einen Winkel von dreißig Grad zueinander bilden. In der Summe entsteht eine radiale Kraftkomponente beim Durchstoßen, die in dieselbe Richtung wie die der Fläche 26 gerichtet ist.

Im Fertigungsprozess werden zunächst jeweils die anteiligen radialen Kraftkomponenten beim Durchstoßen des Verschlusses berechnet. Der Anteil der Kraftkomponente in radialer Richtung kann mittels des Neigungswinkels der jeweiligen Fläche ermittelt werden. Die so ermittelten Kraftkomponenten werden vektoriell addiert und die Neigungswinkel und Oberflächengrößen der Flächen 22, 26, 28, 30 so gewählt, dass sich die Kraftkomponenten zu Null addieren.

Der so ermittelte Anschliff wird im Folgenden durch Durchstoßversuche überprüft. Einerseits wurden die nicht angeschliffenen Oberflächen im Bereich der Spitze 8 nicht berücksichtigt, andererseits haben diese, gezogenen, nicht geschliffenen Oberflächen ein vollkommen anderes Reibungsverhalten als die Flächen 22, 26, 28 und 30. In den Durchstoßversuchen werden die Anschliffe derart optimiert, dass die in der Praxis entstehenden Kraftkomponenten, die von den theoretisch ermittelten abweichen, sich tatsächlich ausgleichen und keine radiale Auslenkung mehr entsteht. Das so ermittelte Anschliffmuster mit Neigung, Oberfläche und Anordnung der Flächen 22, 26, 28, 30 wird dann direkt zur Herstellung weiterer Hohlnadeln 1 verwendet.

Die folgenden FIG 4, FIG 5 und FIG 6 zeigen weitere Darstellungen der Hohlnadel 1 aus verschiedenen Ansichten und werden nur noch hinsichtlich ihrer Besonderheiten erläutert.

FIG 4 zeigt eine seitliche Aufsicht der Hohlnadel 1. Die Flächen 22 und 26 sind hier im Profil erkennbar, die Fläche 30 in der Aufsicht. Die Fläche 28 ist verdeckt. Die Fläche 26 ist um einunddreißig Grad gegenüber der Achse 18 geneigt, die Fläche 22, die den Austritt 24 umschließt, lediglich um neunzehn Grad. Die Spitze 8 ist 0,11 mm von der Achse 18 versetzt. In axialer Richtung erstreckt sich die Fläche 22 auf einer Höhe von 1,41 mm.

FIG 4 zeigt die Schnittebene V-V, deren Ansicht in FIG 5 dargestellt ist. Ähnlich zur FIG 3 zeigt auch FIG 5 die Flächen 22, 26, 28, 30 und deren Neigungen. Hier wird jedoch insbesondere deutlich, dass die Mindestwanddicke 34 eingehalten werden muss, so dass die Fläche 26 nicht tiefer angeschliffen werden kann.

FIG 6 zeigt die rückseitige Aufsicht der Hohlnadel 1 mit den Flächen 26, 28 und 30. Der Austritt 24 und die Fläche 22 sind verdeckt. Erkennbar ist die ellipsenartige Form der Fläche 26. Dargestellt ist auch das Profil der Spitze 8, die durch den Anschliff in der Art einer gebogenen Schneide ausgebildet ist.

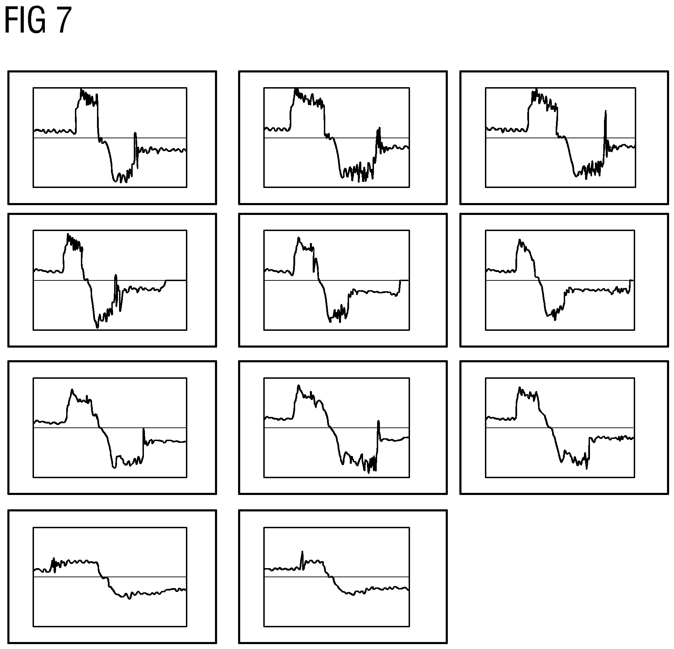

FIG 7 zeigt schließlich eine grafische Darstellung von Versuchsprotokollen mehrerer Anschliffe im beschriebenen Optimierungsprozess. Dabei ist für drei verschiedene Anschliffe (waagerecht) und vier verschiedene Blutentnahmeröhrchen mit unterschiedlichen Verschlussvorrichtungen (senkrecht) jeweils beispielhaft die Stromaufnahme (jeweils linke Y-Achse) des Verfahrmotors gegen die Zeit aufgetragen. Die Stromaufnahme entspricht einer aufzuwendenden Kraft (jeweils rechte Y-Achse) beim Durchstoßen des Verschlusses. Die Hohlnadel 1 wird nach unten gefahren und anschließend wieder zurück in die Ausgangsposition, d.h. der erste positive Anstieg entsteht beim Durchstoßen, der negative Ausschlag beim Entfernen der Hohlnadel 1 aus dem Verschluss.

FIG 7 zeigt, dass Kräfte bis zu 60 N wirken. Für jeden Durchstoßversuch wird die radiale Auslenkung gemessen, und die Anschliffe der Flächen 22, 26, 28, 30 werden entsprechend angepasst, so dass sich die radialen Kräfte ausgleichen.

### Bezugszeichenliste

- 1: Hohlnadel
- 2, 4, 6: Abschnitt
- 8: Spitze
- 10: Vertiefung
- 12: Hohlkanal
- 14: Ende
- 16: Biegung
- 18: Achse
- 20: Biegeradius
- 22: Fläche
- 24: Austritt
- 26, 28, 30: Fläche
- 32: Schnittlinie
- 34: Mindestwanddicke
- II-II: Schnittebene
- IV-IV: Bereich
- C: Symmetrieebene
- V-V: Schnittebene

## Patentansprüche

1. Hohlnadel (1) zum Durchstoßen einer Verschlussvorrichtung eines Probengefäßes, wobei die Hohlnadel (1) eine gerade zylindrische Grundform mit einer Spitze (8) zum Durchstoßen der Verschlussvorrichtung aufweist, und die im Bereich der Spitze (8) gebogen ist und mindestens vier Flächen (22, 26, 28, 30) aufweist deren Oberflächengrößen und Winkel zur Achse (18) der Hohlnadel (1) derart gewählt sind, dass sich die an den jeweiligen Flächen (22, 26, 28, 30) bei einer axialen Bewegung in ein elastisches Material resultierenden radialen Kraftkomponenten gegenseitig aufheben und dass die Normalen zweier der Flächen (22, 26) mit der Achse (18) der Hohlnadel (1) in einer Ebene liegen und wobei der Hohlkanal (12) der Hohlnadel (1) im Bereich der Spitze (8) gebogen ist und eine der Flächen (22) den Austritt (24) des Hohlkanals (12) der Hohlnadel (1) umschließt.

2. Hohlnadel (1) nach Anspruch 1, die einen sich verbreiternden axialen Abschnitt (6) umfasst, der eine axiale Vertiefung (10) aufweist.

3. Hohlnadel (1) nach einem der vorhergehenden Ansprüche, bei der das Profil der Spitze (8) in der Art einer gebogenen Schneide ausgebildet ist.

4. Probenpipettor für ein automatisches Analysegerät mit einer Hohlnadel (1) nach einem der vorherigen Ansprüche.

5. Automatisches Analysegerät mit einem Probenpipettor gemäß Anspruch 4.

6. Verfahren zum Herstellen einer Hohlnadel (1)gemäß Anspruch 1, wobei im Bereich der Spitze (8) mindestens vier Flächen (22, 26, 28, 30) hinsichtlich ihrer Winkel zur Achse (18) der Hohlnadel (1) und der Größe ihrer Oberflächen derart eingebracht werden, dass sich die an den jeweiligen Flächen (22, 26, 28, 30) bei einer axialen Bewegung in ein elastisches Material resultierenden radialen Kraftkomponenten gegenseitig aufheben und dass die Normalen zweier der Flächen (22, 26) mit der Achse (18) der Hohlnadel (1) in einer Ebene liegen und wobei eine der Flächen (22) den Austritt (24) des Hohlkanals (12) der Hohlnadel (1) umschließt.

7. Verfahren nach Anspruch 6, wobei die mindestens vier Flächen (22, 26, 28, 30) durch Anschleifen der Oberfläche im Bereich der Spitze (8) eingebracht werden.

## Claims

1. Hollow needle (1) for piercing a sealing device on a sample vessel, wherein the hollow needle (1) has a straight cylindrical basic shape with a tip (8) for piercing the sealing device and said needle being bent and having at least four faces (22, 26, 28, 30) in the region of the tip (8), of which faces the surface sizes and angles to the axis (18) of the hollow needle (1) are selected such that the radial force components resulting at the respective faces (22, 26, 28, 30) during an axial movement into an elastic material cancel one another and that the normals of two of the faces (22, 26) lie in a plane with the axis (18) of the hollow needle (1) and wherein the hollow channel (12) in the hollow needle (1) is bent in the region of the tip (8) and one of the faces (22) encloses the outlet (24) of the hollow channel (12) in the hollow needle (1).

2. Hollow needle (1) according to Claim 1, which comprises a widening axial portion (6), which has an axial recess (10) .

3. Hollow needle (1) according to one of the preceding claims, in which the profile of the tip (8) is designed in the style of a bent blade.

4. Sample pipettor for an automated analysis instrument with a hollow needle (1) according to one of the preceding claims.

5. Automated analysis instrument with a sample pipettor according to Claim 4.

6. Method for producing a hollow needle (1) according to Claim 1, wherein at least four faces (22, 26, 28, 30) are introduced in the region of the tip (8) with respect to their angles to the axis (18) of the hollow needle (1) and the size of their surfaces such that the radial force components resulting at the respective faces (22, 26, 28, 30) during an axial movement into an elastic material cancel one another and that the normals of two of the faces (22, 26) lie in a plane with the axis (18) of the hollow needle (1) and wherein one of the faces (22) encloses the outlet (24) of the hollow channel (12) in the hollow needle (1).

7. Method according to Claim 6, wherein the at least four faces (22, 26, 28, 30) are introduced by grinding the surface in the region of the tip (8).

## Revendications

1. Aiguille (1) creuse pour transpercer un dispositif de fermeture d'un récipient à échantillon, l'aiguille (1) creuse ayant une forme de base cylindrique droite, en ayant une pointe (8) pour transpercer le dispositif de fermeture, et étant incurvée dans la région de la pointe (8) et ayant au moins quatre faces (22, 26, 28, 30), dont les dimensions de surface et l'angle par rapport à l'axe (18) de l'aiguille (1) creuse sont choisis de manière à ce que les composantes de force radiales sur les faces (22, 26, 28, 30) respectives résultant d'un déplacement axial dans une matière élastique se compensent et de manière à ce que les normales à deux des faces (22, 26) se trouvent, avec l'axe (18) de l'aiguille (1) creuse, dans un plan et dans laquelle le canal (12) creux de l'aiguille (1) creuse est incurvé dans la région de la pointe (8) et l'une des faces (22) entoure la sortie (24) du canal (12) creux de l'aiguille (1) creuse.

2. Aiguille (1) creuse suivant la revendication 1, qui comprend un tronçon (6) axial s'élargissant, qui a une cavité (10) axiale.

3. Aiguille (1) creuse suivant l'une des revendications précédentes, dans lequel le profil de la pointe (8) est constitué à la manière d'un tranchant incurvé.

4. Pipette à échantillon pour un appareil d'analyse automatique, ayant une aiguille (1) creuse suivant l'une des revendications précédentes.

5. Appareil d'analyse automatique ayant une pipette de prélèvement suivant la revendication 4.

6. Procédé de fabrication d'une aiguille (1) creuse suivant la revendication 1, dans lequel on ménage, dans la région de la pointe (8), au moins quatre faces (22, 26, 28, 30), du point de vue de leur angle avec l'axe (18) de l'aiguille (1) creuse et de la dimension de leur surface de manière à ce que les composantes de force radiale sur les faces (22, 26, 28, 30) respectives lors d'un déplacement axial dans une matière élastique se compensent mutuellement et en ce que les normales à deux des faces (22, 26) soient dans un plan avec l'axe (18) de l'aiguille (1) creuse
et dans lequel l'une des faces (22) entoure la sortie (24) du canal (12) creux de l'aiguille (1) creuse.

7. Procédé suivant la revendication 6, dans lequel on ménage les au moins quatre faces (22, 26, 28, 30) par rectification de la surface dans la région de la pointe (8).
